# EUROPÄISCHE PATENTANMELDUNG

(11) **EP 1 925 295 A1**
(43) Veröffentlichungstag der Anmeldung: **28.05.2008**
(21) Anmeldenummer: 06124580.9
(22) Anmeldetag: 22.11.2006
(51) Int. Cl.: A61K 9/14

(54) **Stabile Pulverformulierung enthaltend ein Anticholinergikum**

(71) Anmelder: Boehringer Ingelheim Pharma GmbH & Co. KG, 55216 Ingelheim am Rhein (DE)
(72) Erfinder: Die Erfindernennung liegt noch nicht vor
(74) Vertreter: Hammann, Heinz

(57) **Zusammenfassung**

Die vorliegende Erfindung betrifft eine sprühgetrocknete Pulverformulierung umfassend Partikel, die die folgenden Komponenten i) bis iii) enthalten:
i) Anticholinergika, insbesondere mindestens eine Verbindung der Formel **1** 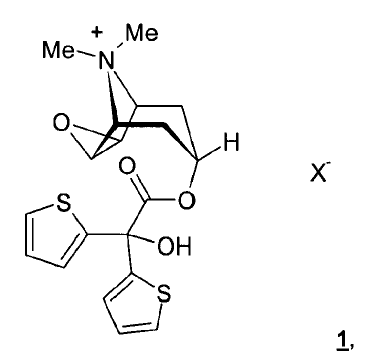 worin
X⁻
ein negativ geladenes Anion bedeutet,

ii) mindestens ein Einbettungsmaterial ausgewählt aus der Gruppe bestehend aus Mono- oder Disacchariden, Oligosacchariden, Polymeren, Zuckeralkoholen und Cholesterin,
iii) ) eine organische, physiologisch unbedenkliche, sterisch anspruchsvolle Säure ausgewählt aus der Gruppe bestehend aus Ascorbinsäure, einer ein-, zwei- oder dreiwertigen Carbonsäure mit Ausnahme der Aminocarbonsäuren, vorzugsweise Fumarsäure, Oxalsäure oder Bernsteinsäure, und einer Frucht- oder Genusssäure, vorzugsweise Citronensäure, Weinsäure, Äpfelsäure, Milchsäure, Essigsäure, α-Hydroxycaprylsäure oder Gluconsäure,
ein Verfahren zu deren Herstellung, sowie die Verwendung einer organischen, physiologisch unbedenklichen, sterisch anspruchsvollen Säure aus der Gruppe bestehend aus Ascorbinsäure, einer ein-, zwei- oder dreiwertigen Carbonsäure, mit Ausnahme der Aminocarbonsäuren, vorzugsweise Fumarsäure, Oxalsäure oder Bernsteinsäure, und einer Frucht- oder Genusssäure, vorzugsweise Citronensäure, Weinsäure, Äpfelsäure, Milchsäure, Essigsäure, α-Hydroxycaprylsäure oder Gluconsäure, zur Stabilisierung einer mittels Sprühtrocknung hergestellten Pulverformulierung enthaltend die obige Verbindung der Formel **1** nach i) und ein geeignetes Einbettungsmaterial ii).

## Beschreibung

Die vorliegende Erfindung betrifft eine sprühgetrocknete Pulverformulierung umfassend Partikel, die die folgenden Komponenten i) bis iii) enthalten:
i) Anticholinergika, insbesondere mindestens eine Verbindung der Formel 1 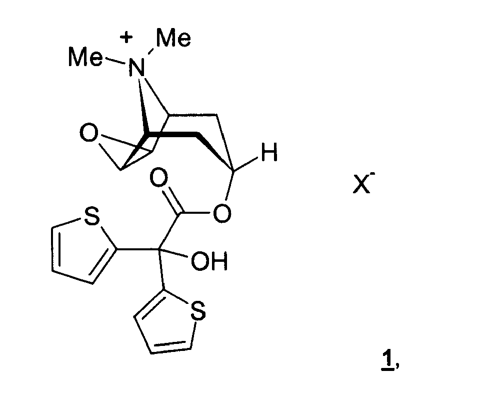 worin
   X ⁻ ein negativ geladenes Anion bedeutet.
ii) mindestens einem Einbettungsmaterial ausgewählt aus der Gruppe bestehend aus Mono- oder Disacchariden, Oligosacchariden, Polymeren, Zuckeralkoholen und Cholesterin,
iii) eine organische, physiologisch unbedenkliche, sterisch anspruchsvolle Säure ausgewählt aus der Gruppe bestehend aus Ascorbinsäure, einer ein-, zwei- oder dreiwertigen Carbonsäure mit Ausnahme der Aminocarbonsäuren, vorzugsweise Fumarsäure, Oxalsäure oder Bernsteinsäure, und einer Frucht- oder Genusssäure, vorzugsweise Citronensäure, Weinsäure, Äpfelsäure, Milchsäure, Essigsäure, α-Hydroxycaprylsäure oder Gluconsäure.

Weiterhin betrifft die Erfindung ein Verfahren zur Herstellung der obigen sprühgetrockneten Pulverformulierung. Außerdem betrifft die Erfindung die Verwendung einer organischen, physiologisch unbedenklichen, sterisch anspruchsvollen Säure ausgewählt aus der Gruppe bestehend aus Ascorbinsäure, einer ein-, zwei- oder dreiwertigen Carbonsäure, mit Ausnahme der Aminocarbonsäuren, vorzugsweise Fumarsäure, Oxalsäure oder Bernsteinsäure, und einer Frucht- oder Genusssäure, vorzugsweise Citronensäure, Weinsäure, Äpfelsäure, Milchsäure, Essigsäure, α-Hydroxycaprylsäure oder Gluconsäure, vorzugsweise der Citronensäure, zur Stablilierung einer mittels Sprühtrocknung hergestellten Pulverformulierung enthaltend
i) Anticholinergika, insbesondere mindestens eine Verbindung der Formel 1 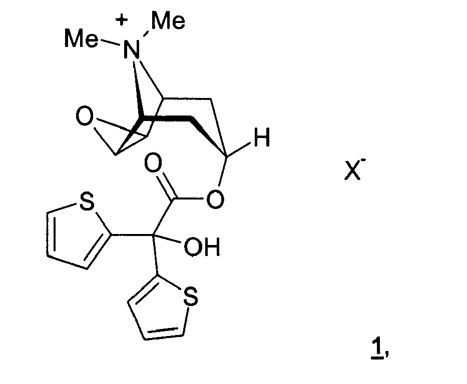 worin
   X ⁻ ein negativ geladenes Anion bedeutet.
ii) mindestens einem Einbettungsmaterial ausgewählt aus der Gruppe bestehend aus Mono- oder Disacchariden, Oligosacchariden, Polymeren, Zuckeralkoholen und Cholesterin.

Verbindungen der Formel **1** sind aus der WO 02/30928 bereits bekannt. Sie besitzen wertvolle pharmakologische Eigenschaften und können als hochwirksame Anticholinergika bei der Therapie von Atemwegserkrankungen, vorzugsweise bei der Therapie entzündlicher und/oder obstruktiver Atemwegserkrankungen, insbesondere bei der Therapie von Asthma oder COPD (chronic obstructive pulmonary disease = chronisch obstruktive Lungenerkrankung) einen therapeutischen Nutzen entfalten.

Geeignete Pulverformulierungen enthaltend mindestens eine Verbindung nach Formel **1** müssen diverse Anforderungen erfüllen:
■ die Pulverformulierungen müssen Partikel enthalten, die "inhalierbar" sind, d.h. die Partikel müssen einen relativ kleinen mittleren Durchmesser haben, vorzugsweise von ≤10 µm um noch "lungenängig" zu sein, d.h. um eine topische Applikation in der Lunge zu ermöglichen
■ die Pulverformulierung muß bei Lagerung bei Raumtemperatur eine ausreichende Stabilität über einen längeren Zeitraum besitzen
■ homogene Verteilung der einzelnen Komponenten i) bis iii) in den Partikeln.

Der mittlere geometrische Durchmesser von Partikeln kann beispielsweise durch ein Laserbeugungsverfahren mit einem Laser der Firma Sympatec (50 mm Brennweite) bei Trockendispergierung (Rodos 3 bar) experimentell bestimmt werden.

Es war folglich Aufgabe der vorliegenden Erfindung, eine Pulverformulierung enthaltend ein Anticholinergikum, insbesondere die Verbindung nach Formel **1**, als Wirkstoff bereitzustellen, die die oben genannten Bedingungen erfüllt.

Für die Herstellung einer Pulverformulierung gibt es beispielsweise die Möglichkeit der Mikronisierung des Wirkstoffs, vorzugsweise mit Hilfe einer Luftstrahlmühle. Bei der Mikronisierung der Verbindung nach Formel **1** haben sich jedoch einige Nachteile ergeben. So weisen z.B. mikronisierte Pulverformulierungen enthaltend eine Verbindung nach Formel **1** als Wirkstoff in der Regel bereits nach kurzer Zeit unter der Einwirkung von Feuchte ein Partikelwachstum auf, so daß die Partikel dann nur noch bedingt "lungengängig" sind. Weiterhin ergibt sich bei der Mikronisierung der Verbindung nach Formel **1** der Nachteil, dass sich hierbei eine wenig homogene Verteilung des Wirkstoffs - d.h. der Verbindung nach Formel **1** - in der Gesamtformulierung ergibt. Die angestrebte therapeutisch wirksame Dosis für die Verbindung nach Formel **1** ist mit ca. 0,1 % Wirkstoff bezogen auf die Gesamtformulierung sehr klein und infolgedessen ist eine gleichmäßige Verteilung dieser geringen Menge Wirkstoff in der Gesamtformulierung durch einfaches Mikronisieren und Mischen kaum möglich.

Eine weitere Möglichkeit zur Herstellung einer Pulverformulierung ist die Herstellung der Formulierung mittels Sprühtrocknung. Hierbei wird eine Lösung aus dem Wirkstoff und einem geeigneten inerten Einbettungsmaterial vorzugsweise mittels einer Düse zerstäubt und anschließend im Heißluftstrom getrocknet. Doch auch bei der Herstellung einer Pulverformulierung enthaltend mindestens eine Verbindung der Formel **1** als Wirkstoff und ein geeignetes Einbettungsmaterial durch Sprühtrocknung wurden massive Probleme bezüglich der chemischen Stabilität der Verbindung nach Formel **1** beobachtet. Wird bei der Sprühtrocknung eine Lösung enthaltend lediglich eine Verbindung nach Formel **1** und ein geeignetes Einbettungsmaterial zerstäubt und anschließend getrocknet, so führt dies zu einer starken chemischen Zersetzung der Verbindung nach Formel **1**.

Überraschenderweise hat sich jedoch ergeben, dass eine sprühgetrocknete Pulverformulierung umfassend Partikel, die die folgenden Komponenten i) bis iii) enthalten:
i) ein Anticholinergikum, vorzugsweise eine Verbindung der Formel **1** 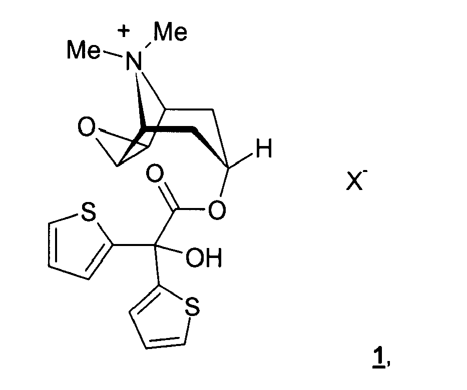 worin
   X ⁻ ein negativ geladenes Anion bedeutet.
ii) mindestens einem Einbettungsmaterial ausgewählt aus der Gruppe bestehend aus Mono- oder Disacchariden, Oligosacchariden, Polymeren, Zuckeralkoholen und Cholesterin,
iii) eine organische, physiologisch unbedenkliche, sterisch anspruchsvolle Säure, vorzugsweise ausgewählt aus der Gruppe bestehend aus Ascorbinsäure, einer ein-, zwei- oder dreiwertigen Carbonsäure mit Ausnahme der Aminocarbonsäuren, vorzugsweise Fumarsäure, Oxalsäure oder Bernsteinsäure, und einer Frucht- oder Genusssäure, vorzugsweise Citronensäure, Weinsäure, Äpfelsäure, Milchsäure, Essigsäure, α-Hydroxycaprylsäure oder Gluconsäure
bei Raumtemperatur (und sogar bei Temperaturen von bis zu 40°C) über einen längeren Zeitraum chemisch und physikalisch stabil sind.

Diese bessere Stabilität wird insbesondere durch die stabilisierende Wirkung der organischen, physiologisch unbedenklichen, sterisch anspruchsvollen Säure, vorzugsweise ausgewählt aus der Gruppe bestehend aus Ascorbinsäure, einer ein-, zwei- oder dreiwertigen Carbonsäure mit Ausnahme der Aminocarbonsäuren, vorzugsweise Fumarsäure, Oxalsäure oder Bernsteinsäure, und einer Frucht- oder Genusssäure, vorzugsweise Citronensäure, Weinsäure, Äpfelsäure, Milchsäure, Essigsäure, α-Hydroxycaprylsäure oder Gluconsäure auf das Anticholinergikum, vorzugsweise auf die Verbindung der Formel **1** verursacht. Weiterhin weist die erfindungsgemäße Pulverformung inhalierbare, d.h. lungengängige Partikelgrößen und außerdem eine homogene Verteilung der Verbindung der Formel **1** in den Partikeln auf. Somit erfüllt die erfindungsgemäße Pulverformulierung sämtliche Anforderungen, die eine Pulverformulierung enthaltend eine Verbindung der Formel **1** als Wirkstoff erfüllen muß.

Die erfindungsgemäße sprühgetrocknete Pulverformulierung enthält als organische, physiologisch unbedenkliche, sterisch anspruchsvolle Säure nach iii) vorzugsweise eine Säure ausgewählt aus der Gruppe bestehend aus Ascorbinsäure, einer ein-, zwei- oder dreiwertigen Carbonsäure (mit Ausnahme der Aminocarbonsäuren),und einer Frucht- oder Genusssäure.

Unter einer ein-, zwei- oder dreiwertigen Carbonsäure im Sinne der Erfindung werden C₂- bis C₁₀-, vorzugsweise C₃-bis C₆-Carbonsäuren mit jeweils einer, zwei oder drei Carboxylgruppen mit Ausnahme der Aminocarbonsäuren verstanden, wobei Fumarsäure, Oxalsäure, Bernsteinsäure, Citronensäure, Weinsäure, Äpfelsäure, Milchsäure, Essigsäure, α-Hydroxycaprylsäure oder Gluconsäure bevorzugt, Citronensäure besonders bevorzugt ist. Unter einer Genuss- oder Fruchtsäure im Sinne der Erfindung werden vorzugsweise die Säuren ausgewählt aus der Gruppe Citronensäure, Weinsäure, Äpfelsäure, Milchsäure, Essigsäure, α-Hydroxycaprylsäure oder Gluconsäure, besonders bevorzugt Citronensäure, verstanden.

In einer weiteren bevorzugten Ausführungsform enthält die sprühgetrocknete Pulverformulierung die organische, physiologisch unbedenkliche, sterisch anspruchsvolle Säure nach iii) in der Konzentration, die notwendig ist, um der sprühfertigen Lösung enthaltend das Anticholinergikum nach i), vorzugsweise eine Verbindung nach Formel **1**, und das mindestens eine Einbettungsmaterial nach ii) einen pH von < 7, vorzugsweise von ≤6, stärker bevorzugt von ≤5, insbesondere von ≤4 zu verleihen.

Besonders bevorzugt enthält die sprühgetrocknete Pulverformulierung als organische, physiologisch unbedenkliche, sterisch anspruchsvolle Säure nach iii) Citronensäure in der Konzentration, die notwendig ist, um der sprühfertigen Lösung enthaltend das Anticholinergikum nach i), vorzugsweise eine Verbindung nach Formel **1**, und das mindestens eine Einbettungsmaterial nach ii) einen pH von < 7, vorzugsweise von ≤6, stärker bevorzugt von ≤5, insbesondere von ≤4 zu verleihen.

Weiterhin hat sich überraschend gezeigt, dass sich auch die Gegenwart des Salzes einer organischen, physiologisch unbedenklichen, sterisch anspruchsvollen Säure zusätzlich zur organischen, physiologisch unbedenklichen, sterisch anspruchsvollen Säure nach iii) ausgesprochen günstig auf die chemische Stabilität der erfindungsgemäßen sprühgetrockneten Pulverformulierung auswirkt. Infolgedessen enthält die oben genannte, erfindungsgemäße sprühgetrocknete Pulverformulierung in einer besonders bevorzugten Ausführungsform zusätzlich das Salz einer organischen, physiologisch unbedenklichen, sterisch anspruchsvollen Säure, vorzugsweise ausgewählt aus der Gruppe bestehend aus Ascorbat, dem Salz einer ein-, zwei- oder dreiwertigen Carbonsäure mit Ausnahme der Salze der Aminocarbonsäuren, vorzugsweise Fumarat, Oxalat oder Succinat, und dem Salz einer Frucht- oder Genusssäure, vorzugsweise Citrat, Tartrat, Malat, Lactat, Acetat, α-Hydroxycapronat oder Gluconat. Besonders bevorzugt ist hierbei Citrat. Dieses zusätzliche Salz einer organischen, physiologisch unbedenklichen, sterisch anspruchsvollen Säure nach iii) ist hierbei vorzugsweise ein Alkalimetallsalz, ein Erdalkalimetallsalz oder ein Zinksalz, vorzugsweise ein Alkalimetallcitrat, ein Erdalkalimetallcitrat oder ein Zinkcitrat, besonders bevorzugt Natriumcitrat, Kaliumcitrat, Magnesiumcitrat, Calciumcitrat oder Zinkcitrat.

Das zusätzliche Salz einer organischen, physiologisch unbedenklichen, sterisch anspruchsvollen Säure ausgewählt aus der Gruppe bestehend aus Ascorbat, dem Salz einer ein-, zwei- oder dreiwertigen Carbonsäure mit Ausnahme der Salze der Aminocarbonsäuren, vorzugsweise Fumarat, Oxalat oder Succinat, und dem Salz einer Frucht- oder Genusssäure, vorzugsweise Citrat, Tartrat, Malat, Lactat, Acetat, α-Hydroxycapronat oder Gluconat wird hierbei vorzugsweise in der Konzentration eingesetzt, dass ein molares Verhältnis des Anticholinergikums nach i), vorzugsweise der Verbindung nach Formel 1, zum Kation des Salzes von 1:1 bis 1:12, vorzugsweise von 1:2 bis 1:10 und insbesondere von 1:3 bis 1:8 ergibt.

Die erfindungsgemäße sprühgetrocknete Pulverformulierung umfasst vorzugsweise eine Verbindung der Formel **1**, worin X ⁻ ein Anion ausgewählt aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, insbesondere Bromid ist.

In einer bevorzugten Ausführungsform umfasst die sprühgetrocknete Pulverformulierung das Anticholinergikum, vorzugsweise die oben definierte Verbindung nach Formel **1** nach i) in einer Konzentration zwischen 0,01 bis 5 %, bezogen auf den Feststoff, stärker bevorzugt in einer Konzentration zwischen 0,02 bis 2 % bezogen auf den Feststoff, insbesondere in einer Konzentration zwischen 0,05 bis 1 % bezogen auf den Feststoff.

In einer weiteren bevorzugten Ausführungsform umfasst die sprühgetrocknete Pulverformulierung als Einbettungsmaterial ii) ein Mono- oder Disaccharid ausgewählt aus der Gruppe bestehend aus Glucose, Fructose, Arabinose, Mannitol, Saccharose, Maltose, Lactose, Cellobiose und Trehalose.

In einer anderen bevorzugten Ausführungsform umfasst die sprühgetrocknete Pulverformulierung als Einbettungsmaterial ii) ein Oligosaccharid ausgewählt aus der Gruppe Oligomaltose, Oligofructose, Cyclodextrine, Dextrane, Dextrine (z.B. Cyclodextrine wie beispielsweise α-Cyclodextrin, β-Cyclodextrin, γ-Cyclodextrin, Methyl-β-Cyclodextrin, Hydroxypropyl-β-Cyclodextrin) und Oligosaccharose.

In einer anderen bevorzugten Ausführungsform umfasst die sprühgetrocknete Pulverformulierung als Einbettungsmaterial ii) ein Polymer ausgewählt aus der Gruppe Inulin, Alginat, Maltodextrin, Stärke, Stärkederivate, Cellulose, Cellulosederivate, PVP (Plasdone), Gelatine, Chitosan, Dextrane, Pektine, Gummi Arabicum, Polylaktide, Poly(laktid-co-glykolide) und Polyvinylalkohole.

In einer anderen bevorzugten Ausführungsform umfasst die sprühgetrocknete Pulverformulierung als Einbettungsmaterial ii) einen Zuckeralkohol ausgewählt aus der Gruppe Mannitol, Xylitol und Sorbitol.

In einer anderen bevorzugten Ausführungsform umfasst die sprühgetrocknete Pulverformulierung als Einbettungsmaterial ii) Cholesterin.

In einer weiteren bevorzugten Ausführungsform umfasst die erfindungsgemäße sprühgetrocknete Pulverformulierung neben den Bestandteilen i) bis iii) auch noch weitere physiologisch unbedenkliche Hilfsstoffe. Als physiologisch unbedenkliche Hilfsstoffe, die zur Darstellung der im Rahmen der erfindungsgemäßen sprühgetrockneten Pulverformulierungen zur Anwendung gelangen können, seien beispielsweise Salze, z.B. Natriumchlorid, Kaliumchlorid etc., insbesondere, aber nicht ausschließlich in Form ihrer Hydrate, Komplexbildner, Geschmacksstoffe, Konservierungsstoffe und Vitamine genannt.

In einer weiteren bevorzugten Ausführungsform umfasst die erfindungsgemäße sprühgetrocknete Pulverformulierung Partikel, die einen mittleren aerodynamischen Durchmesser von ≤15 µm, vorzugsweise von ≤10 µm, insbesondere von ≤5 µm besitzen. Besonders bevorzugt sind erfindungsgemäße sprühgetrocknete Pulverformulierungen, die Partikel enthalten, die "inhalierfähig" sind. "Inhalierfähige Partikel" oder "lungengängige Partikel" bedeuten im Rahmen der vorliegenden Erfindung, dass diese Partikel einen mittleren aerodynamischen Durchmesser besitzen, der klein genug ist, um eine topische Applikation in der Lunge zu erreichen. Dies ist insbesondere dann der Fall, wenn die Partikel einen mittleren aerodynamischen Durchmesser ≤10 µm besitzen.

Der mittlere aerodynamische Durchmesser (MMAD) kann experimentell nach dem Kaskadenimpaktor-Verfahren experimentell bestimmt werden, welches in der European Pharmacopeia, im Supplement 2000 zur Bestimmung des MMAD beschrieben wird.

Die Applikation der Verbindung nach Formel **1** erfolgt hierbei vorzugsweise auf inhalativem Wege. Hierbei können neben Inhalationslösungen auch geeignete Inhalationspulver, die vorzugsweise in geeignete Kapseln (Inhaletten) abgefüllt mittels entsprechender Pulverinhalatoren appliziert werden, zum Einsatz kommen.

Die erfindungsgemäßen Inhalationspulver können beispielsweise mittels Inhalatoren appliziert werden, die eine einzelne Dosis aus einem Vorrat mittels einer Meßkammer (z.B. gemäß US 4570630A) oder über andere apparative Vorrichtungen (z.B. gemäß DE 36 25 685 A) dosieren.

Alternativ dazu und erfindungsgemäß von gleichrangiger Bedeutung, können die erfindungsgemäßen Inhalationspulver auch mittels Inhalatoren appliziert werden, die das Inhalationspulver in mehreren, einzeln verpackten Dosen enthalten (Pre-Metered Dry Powder Inhaler). Die mehreren, einzeln verpackten Dosen können hierbei in Form eines Multi-Dosis-Blisters und insbesondere in Form einer Kreisscheibe, die in zirkulär angeordneten Vertiefungen mehrere Pulver-Einzeldosen aufnehmen kann, vorhanden sein. Alternativ können die mehreren, einzeln verpackten Dosen auch in Form eines Blisterstreifens angeordnet sein.

Alternativ dazu und erfindungsgemäß von gleichrangiger Bedeutung, können die erfindungsgemäßen Inhalationspulver auch in Kapseln abgefüllt werden, die in Inhalatoren wie beispielsweise in der WO 94/28958 beschrieben, zur Anwendung gelangen.

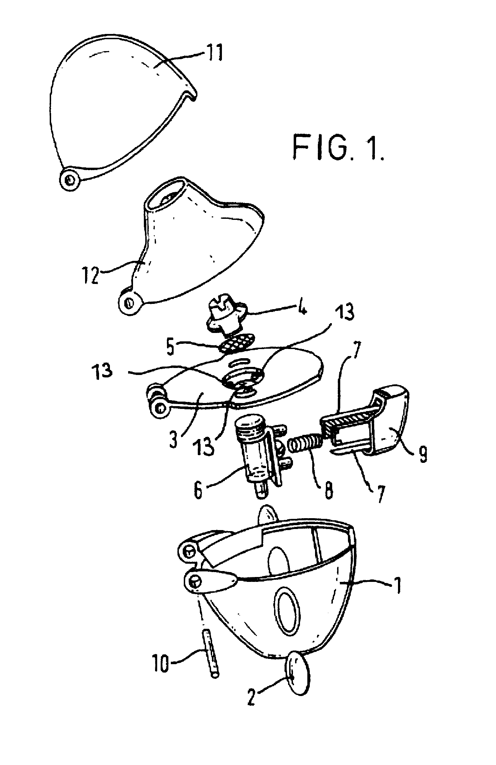

Vorzugsweise werden die das erfindungsgemäße Inhalationspulver enthaltenden Kapseln mit einem Inhalator appliziert, wie er in Figur 1 dargestellt ist.
Dieser Inhalator ist gekennzeichnet durch ein Gehäuse 1, enthaltend zwei Fenster 2, ein Deck 3, in dem sich Lufteinlaßöffnungen befinden und welches mit einem über ein Siebgehäuse 4 befestigten Sieb 5 versehen ist, eine mit Deck 3 verbundene Inhalationskammer 6, an der ein mit zwei geschliffenen Nadeln 7 versehener, gegen eine Feder 8 beweglicher Drücker 9 vorgesehen ist, ein über eine Achse 10 klappbar mit dem Gehäuse 1, dem Deck 3 und einer Kappe 11 verbundenes Mundstück 12, sowie Luftdurchlaßlöcher 13 zur Einstellung des Strömungswiderstandes.

In weiteren bevorzugten Ausführungsform wird die erfindungsgemäße Pulverformulierung in einem Inhalator nach US 5,590,645 angewendet. Auf US 5,590,645 wird hiermit vollinhaltlich Bezug genommen. US 5590645 beschreibt eine Inhalationsvorrichtung zur Verwendung einer Medikamentenverpackung, in der mindestens ein Container für eine pharmazeutische Pulver-Zusammensetzung durch zwei abziehbare Papiere definiert ist.

In weiteren bevorzugten Ausführungsform wird die erfindungsgemäße Pulverformulierung in einem Inhalator nach US 4,627,432 angewendet.
Auf US 4,627,432 wird hiermit vollinhaltlich Bezug genommen. US 4627432 beschreibt einen Inhalator zur Verabreichung von Medikamenten an Patienten, der ein Gehäuse enthaltend eine zylindrische Kammer enthält., Eine Halterung wird innerhalb der Kammer installiert, um einen Träger, z.B. eine Blisterpackung aufzunehmen. Die Blisterverpackung umfasst eine Vielzahl an Containern oder von Blistern, die in einem Kreis angeordnet sind. Wenn der Blisterpack von der Halterung aufgenommen wurde, befinden sich seine Blister in Löchern in der Halterung. Ein Kolben wird so angebracht, dass dieser in die Kammer durch das Loch eindringen kann, wobei ein Blister, der sich darin befindet, geöffnet werden kann. Wenn der Blister geöffnet wird, kann das Medikament vom Patienten durch Inhalation durch das Mundstück entnommen werden. Ein externes Element wird genutzt, um die Halterung zu rotieren, so dass der nächste Blister mit dem Kolben in Kontakt treten kann. Luft kann in die Kammer eintreten durch ein Loch in der Abdeckung, die entfernbar ist, um das Laden der Blisterpackung in die Kammer in die Halterung zu erlauben.

In einer weiteren bevorzugten Ausführungsform wird die erfindungsgemäße Pulverformulierung in einem Inhalator gemäß WO 95/16483 angewendet..Auf WO 95/16483 wird hiermit vollinhaltlich Bezug genommen. WO 95/16483 beschreibt einen Inhalator zur Abgabe von Dosen einer pharmazeutischen Pulver-Zusammensetzung, der ein Gehäuse mit einem zylindrischen Container beinhaltet. Der Container hat mehrere helikal angeordnete Kompartmente, die alle eine Dosis der pharmazeutischen Zusammensetzung beherbergen. Um die pharmazeutische Zusammensetzung aus einem Kompartment zu entlassen, muß dieses Kompartment in den Luftweg des Inhalators mit Hilfe eines Indizierungsmechansismus gebracht werden und der Anwender saugt am Mundstück des Gehäuses, wobei dieses Mundstück mit dem Luftauslass des Luftweges kommuniziert. Der Luftfluss durch den Luftweg entlässt die Einzeldosis des Materials. Der Container kann eine austauschbare Kartusche darstellen.

In einer weiteren bevorzugten Ausführungsform wird die erfindungsgemäße Pulverformulierung in einem Inhalator gemäß WO 95/31238 angewendet..Auf WO 95/31238 wird hiermit vollinhaltlich Bezug genommen.
WO 95/31238 beschreibt einen Inhalator zur Abgabe von Einzeldosen einer pharmazeutischen Pulver-Zusammensetzung, der ein Gehäuse zur Aufnahme eines Containers hat, wobei der Container eine Vielzahl von versiegelten Öffnungen enthaltend individuell verkapselte Dosen eines Medikaments enthält. Der Container kann relativ zum Gehäuse bewegt werden, um jede Öffnung nacheinander in den Luftweg zu bringen, der mit dem Mundstück kommuniziert. Der Inhalator enthält eine Stechvorrichtung, z.B. einen Bolzen, der in eine ausgewählte Öffnung inseriert werden kann, um die entsprechende Versiegelung aufzubrechen. Die Konfiguration und Bewegung des Bolzens sind so koordiniert, dass bei diesem Vorgang nahezu kein Pulver ausgestoßen wird.

In einer weiteren bevorzugten Ausführungsform wird die erfindungsgemäße Pulverformulierung in einem Inhalator gemäß WO 02/26302 angewendet. Auf WO 02/26302 wird hiermit vollinhaltlich Bezug genommen. WO 02/26302 beschreibt einen Inhalator zur Abgabe von Einzeldosen einer pharmazeutischen Pulver-Zusammensetzung, der einen Luftweg besitzt, durch den die Dosis von einer Ejektionszone bis zum Auslass des Luftweges wandert. Der Luftweg hat eine Einlassvorrichtung, die so angeordnet ist, dass sie eine Lufttasche bildet, die durch einen Teil des Luftweges fließt und sich von der Ejektionszone bis zum Auslass ausdehnt. Die Lufttasche umgibt die besagte Dosis und verhindert so, dass diese auf die Wände des Luftweges auftrifft. Dadurch wird die Akkumulation des Materials auf den Wänden des Luftweges reduziert und dadurch wird die Konsistenz der Inhalator-Performance verbessert. Vorzugsweise beinhaltet die Einlassvorrichtung einen Hals zur Herstellung eines Stroms aus schnell fließender Luft, wodurch eine Zone geringen Drucks vor der Ejektionszone gebildet wird, was die Ejektion der Dosis erleichtert.

In einer weiteren bevorzugten Ausführungsform wird die erfindungsgemäße Pulverformulierung in einem Inhalator gemäß WO 05/002654 angewendet. Auf WO 05/002654 wird hiermit vollinhaltlich Bezug genommen.

WO 05/002654 beschreibt einen Inhalator zur Abgabe individueller Einzeldosen einer pharmazeutischen Pulver-Zusammensetzung aus entsprechenden Taschen eines scheibenförmigen Trägers durch die von außen durchgeführte Zerstörung einer Abdeckfolie durch Druck auf der gegenüberliegenden Oberfläche. Der Inhalator weist hierbei entsprechend individuelle Deaggregationszugänge für jede Tasche, aufgespaltenen Luftströme, die ein verbessertes Aufströmen der pharmazeutischen Zusammensetzung erlauben, einen Schalt-Mechanismus für die äußere Zerstörung der Taschen und einen Zähler für die einzelnen Dosen auf.

Die Erfindung betrifft weiterhin ein Verfahren zur Herstellung einer Pulverformulierung, die Partikel enthält, umfassend die folgenden Schritte:
a) Bereitstellen einer Lösung umfassend die Komponenten i) bis iii):
   i) ein Anticholinergikum, vorzugsweise eine Verbindung der Formel **1** 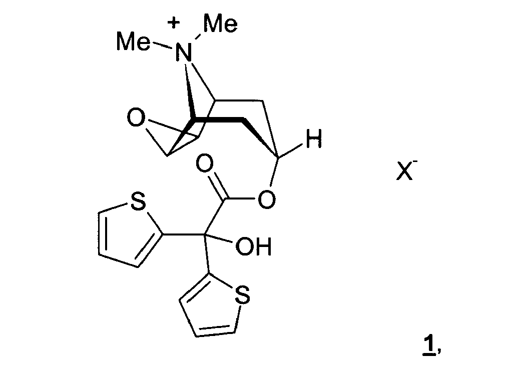 worin
      X ⁻ ein negativ geladenes Anion bedeutet.
   ii) mindestens einem Einbettungsmaterial ausgewählt aus der Gruppe bestehend aus Mono- oder Disacchariden, Oligosacchariden, Polymeren, Zuckeralkoholen und Cholesterin,
   iii) eine organische, physiologisch unbedenkliche, sterisch anspruchsvolle Säure, vorzugsweise ausgewählt aus der Gruppe bestehend aus Ascorbinsäure, einer ein-, zwei- oder dreiwertigen Carbonsäure mit Ausnahme der Aminocarbonsäuren, vorzugsweise Fumarsäure, Oxalsäure oder Bernsteinsäure, und einer Frucht- oder Genusssäure, vorzugsweise Citronensäure, Weinsäure, Äpfelsäure, Milchsäure, Essigsäure, α-Hydroxycaprylsäure oder Gluconsäure
   in einem geeigneten Lösungsmittel;
b) Zerstäuben der Lösung aus Schritt a) in einem Heißluftstrom
c) Trocknen der zerstäubten Lösung aus Schritt b) zu einem Pulver enthaltend Partikel
d) Abscheidung des Pulvers aus Schritt c) mittels Zyklon oder (und) Filter

Für das erfindungsgemäße Verfahren kann jede gängige Sprühtrocknungsvorrichtung verwendet werden, bei dem die Schritte a) bis d) durchgeführt werden.

In Schritt a) wird eine Lösung aus den Komponenten i) bis iii) in einem geeigneten Lösungsmittel bereitgestellt.

Vorzugsweise bedeutet X ⁻ in der Verbindung der Formel **1** ein Anion ausgewählt aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, insbesondere Bromid.

Die Lösung aus Schritt a) enthält hierbei als organische, physiologisch unbedenkliche, sterisch anspruchsvolle Säure nach iii) vorzugsweise eine Säure ausgewählt aus der Gruppe bestehend aus Ascorbinsäure, einer ein-, zwei- oder dreiwertigen Carbonsäure mit Ausnahme der Aminocarbonsäuren, und einer Frucht- oder Genusssäure.

Unter einer ein-, zwei- oder dreiwertigen Carbonsäure im Sinne der Erfindung werden C₂- bis C₁₀-, vorzugsweise C₃-bis C₆-Carbonsäuren mit jeweils einer, zwei oder drei Carboxylgruppen mit Ausnahme der Aminocarbonsäuren verstanden, wobei Fumarsäure, Oxalsäure, Bernsteinsäure, Citronensäure, Weinsäure, Äpfelsäure, Milchsäure, Essigsäure, α-Hydroxycaprylsäure oder Gluconsäure bevorzugt, Citronensäure besonders bevorzugt ist.

Unter einer Genuss- oder Fruchtsäure im Sinne der Erfindung werden vorzugsweise die Säuren ausgewählt aus der Gruppe Citronensäure, Weinsäure, Äpfelsäure, Milchsäure, Essigsäure, α-Hydroxycaprylsäure oder Gluconsäure, besonders bevorzugt Citronensäure, verstanden.

In einer weiteren bevorzugten Ausführungsform des erfindungsgemäßen Verfahrens wird der Lösung aus Schritt a), die bereits das Anticholinergikum nach i), vorzugsweise eine Verbindung nach Formel **1**, und mindestens ein Einbettungsmaterial nach ii) enthält, so viel der organischen, physiologisch unbedenklichen, sterisch anspruchsvollen Säure nach iii) zugegeben, dass die Lösung vor dem Zerstäuben gemäß Schritt b) einen pH von < 7, vorzugsweise von ≤6, stärker bevorzugt von ≤5, insbesondere von ≤4 besitzt.

In einer besonders bevorzugten Ausführungsform des erfindungsgemäßen Verfahrens wird der Lösung aus Schritt a), die bereits das Anticholinergikum nach i), vorzugsweise eine Verbindung nach Formel **1**, und mindestens ein Einbettungsmaterial nach ii) enthält, so viel der Citronensäure zugegeben, dass die Lösung vor dem Zerstäuben gemäß Schritt b) einen pH von < 7, vorzugsweise von ≤6, stärker bevorzugt von ≤5, insbesondere von ≤4 besitzt.

In einer weiteren besonders bevorzugten Ausführungsform enthält die Lösung aus Schritt a) zusätzlich zu den Komponenten i) bis iii) zusätzlich das Salz einer organischen, physiologisch unbedenklichen, sterisch anspruchsvollen Säure nach iii), die vorzugsweise ausgewählt ist aus der Gruppe bestehend aus Ascorbat, dem Salz einer ein-, zwei- oder dreiwertigen Carbonsäure mit Ausnahme der Salze der Aminocarbonsäuren, vorzugsweise Fumarat, Oxalat oder Succinat, und dem Salz einer Frucht- oder Genusssäure, vorzugsweise Citrat, Tartrat, Malat, Lactat, Acetat, α-Hydroxycapronat oder Gluconat. Besonders bevorzugt ist hierbei Citrat. Dieses zusätzliche Salz einer organischen, physiologisch unbedenklichen, sterisch anspruchsvollen Säure nach iii) ist hierbei vorzugsweise ein Alkalimetallsalz, ein Erdalkalimetallsalz oder ein Zinksalz, vorzugsweise ein Alkalimetallcitrat, ein Erdalkalimetallcitrat oder ein Zinkcitrat, besonders bevorzugt Natriumcitrat, Kaliumcitrat, Magnesiumcitrat, Calciumcitrat und Zinkcitrat.

Vorzugsweise wird das Verhältnis der organischen, physiologisch unbedenklichen, sterisch anspruchsvollen Säure, vorzugsweise Citronensäure, zum Salz der organischen, physiologisch unbedenklichen, sterisch anspruchsvollen Säure, vorzugsweise Citrat, in der Lösung derart eingestellt, dass die in Schritt a) hergestellte Lösung enthaltend ein Anticholinergikum nach i), vorzugsweise eine Verbindung nach Formel **1** und mindestens ein Einbettungsmaterial nach ii) einen pH von < 7, vorzugsweise von ≤6, insbesondere von ≤5, stärker bevorzugt von ≤4 besitzt.

Vorzugsweise wird in Schritt a) eine solche Konzentration des Salzes der organischen, physiologisch unbedenklichen, sterisch anspruchsvollen Säure eingesetzt, dass sich ein molares Verhältnis des Anticholinergikums nach i), insbesondere der Verbindung nach Formel **1**, zum Kation des Salzes von 1:1 bis 1:12, vorzugsweise von 1:2 bis 1:10 und insbesondere von 1:3 bis 1:8 ergibt.

Vorzugsweise wird als Einbettungsmaterial ii) ein Mono- oder Disaccharid ausgewählt aus der Gruppe bestehend aus Glucose, Saccharose, Fructose, Maltose, Lactose, Cellobiose und Trehalose, ein Oligosaccharid ausgewählt aus der Gruppe Oligomaltose, Oligofructose, Cyclodextrine, Dextrine und Oligosaccharose, ein Polymer ausgewählt aus der Gruppe Inulin, Alginat, Maltodextrin, Stärke, Stärkederivate, Cellulose, Cellulosederivate, PVP (Plasdone), Gelatine, Chitosan, Dextrane, Pektine, Gummi Arabicum, Polylaktide, Poly(laktid-co-glykolide) und Polyvinylalkohole, ein Zuckeralkohol ausgewählt aus der Gruppe Mannitol, Xylitol und Sorbitol oder Cholesterin eingesetzt. Besonders bevorzugt als Einbettungsmaterial ii) sind jedoch Lactose, Trehalose und Mannitol, insbesondere Lactose.

Vorzugsweise wird die Verbindung der Formel **1** nach i) in einer Konzentration von 0,04g/100ml bis 0,4g/100ml, das Einbettungsmaterial nach ii) in einer Konzentration von 5g/100ml bis 15g/100ml im Lösungsmittel gelöst und die organische, physiologisch unbedenkliche, sterisch anspruchsvolle Säure nach iii) in einer solchen Konzentration zugegeben, dass ein pH von < 7, vorzugsweise von ≤6, stärker bevorzugt von ≤5, insbesondere von ≤4 eingestellt wird.

Als Lösungsmittel kann Wasser, jedes geeignete organische Lösungmittel, ein Wasser/organisches Lösungsmittel-Gemisch und ein Gemisch aus verschiedenen organischen Lösungsmitteln verwendet werden. Bevorzugt ist die Verwendung von Wasser, Ethanol und einem Ethanol/Wasser-Gemisch als Lösungsmittel.

In einer besonders bevorzugten Ausführungsform des erfindungsgemäßen Verfahrens erfolgt das Zerstäuben der Lösung aus Schritt a) in Schritt b) mittels einer Düse. Diese Düse wird vorzugsweise durch Flüssigkeitsdruck oder Pressluft bzw. Inertgas betrieben. Der Zerstäubungsdruck wird derart gewählt, dass Tropfengrößen im Bereich von <20 µm entstehen. Die Tröpfchengrößen der zerstäubten Lösung können beispielsweise mit Hilfe eines Laserbeugungsverfahrens mit einem Laser der Firma Sympatec (50 mm Brennweite, Mie-Auswertung) experimentell bestimmt werden. Vorzugsweise besitzt der Heißluftstrom aus Schritt b) Temperaturen zwischen 100 und 350°C, insbesondere zwischen 120 und 200°C.

In einer weiteren bevorzugten Ausführungsform des erfindungsgemäßen Verfahrens entstehen nach dem Trocknen in Schritt c) sprühgetrocknete Partikel, die einen mittleren aerodynamischen Durchmesser von ≤15 µm, vorzugsweise von ≤10 µm, insbesondere von ≤5 µm besitzen. Besonders bevorzugt ist ein erfindungsgemäßes Verfahren, bei dem nach dem Trocknen in Schritt c) sprühgetrocknete Partikel entstehen, die "inhalierfähig" sind, d.h. die einen Durchmesser haben, der klein genug ist, um eine topische Applikation in der Lunge zu bewirken.

Die Erfindung betrifft weiterhin eine sprühgetrocknete Pulverformulierung enthaltend Partikel, die durch eines der oben genannten erfindungsgemäßen Verfahren erhältlich ist.

Die Erfindung betrifft weiterhin die Verwendung einer organischen, physiologisch unbedenklichen, sterisch anspruchsvollen Säure nach iii) vorzugsweise aus der Gruppe bestehend aus Ascorbinsäure, einer ein-, zwei- oder dreiwertigen Carbonsäure mit Ausnahme der Aminocarbonsäuren, vorzugsweise Fumarsäure, Oxalsäure oder Bernsteinsäure, und einer Frucht- oder Genusssäure, vorzugsweise Citronensäure, Weinsäure, Äpfelsäure, Milchsäure, Essigsäure, α-Hydroxycaprylsäure oder Gluconsäure zur Stabilisierung einer mittels Sprühtrocknung hergestellten Pulverformulierung enthaltend die folgenden Komponenten i) bis ii):
i) ein Anticholinergikum, vorzugsweise eine Verbindung der Formel **1** 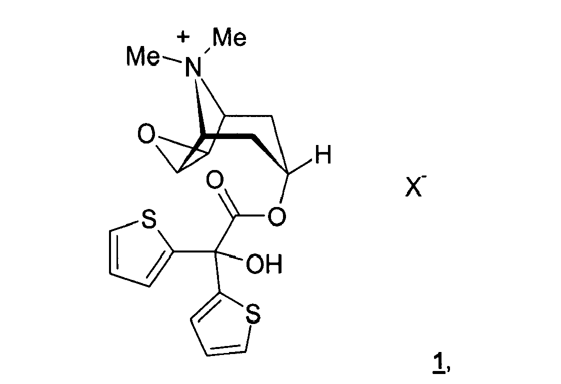 worin
   X ⁻ ein negativ geladenes Anion bedeutet.
ii) mindestens ein Einbettungsmaterial ausgewählt aus der Gruppe bestehend aus Mono- oder Disacchariden, Oligosacchariden, Polymeren, Zuckeralkoholen und Cholesterin.

Besonders bevorzugt wird die organische, physiologisch unbedenkliche, sterisch anspruchsvolle Säure nach iii) in einer solchen Konzentration in der zu sprühtrocknenden Lösung eingesetzt, dass sich in der sprühfertigen Lösung enthaltend das Anticholinergikum nach i), vorzugsweise eine Verbindung nach Formel **1**, und mindestens ein Einbettungsmaterial nach ii) ein pH von < 7, vorzugsweise von ≤6, stärker bevorzugt von ≤ 5, insbesondere von ≤4 ergibt.

In einer weiteren besonders bevorzugten Ausführungsform wird das Salz einer organischen, physiologisch unbedenklichen, sterisch anspruchsvollen Säure nach iii), vorzugsweise ausgewählt aus der Gruppe bestehend aus Ascorbat, dem Salz einer ein-, zwei- oder dreiwertigen Carbonsäure mit Ausnahme der Salze der Aminocarbonsäuren, vorzugsweise Fumarat, Oxalat oder Succinat, und dem Salz einer Frucht- oder Genusssäure, vorzugsweise Citrat, Tartrat, Malat, Lactat, Acetat, α-Hydroxycapronat oder Gluconat zur Stabilisierung einer mittels Sprühtrocknung hergestellten Pulverformulierung enthaltend die folgenden Komponenten i) bis ii):
i) eine Verbindung der Formel **1** 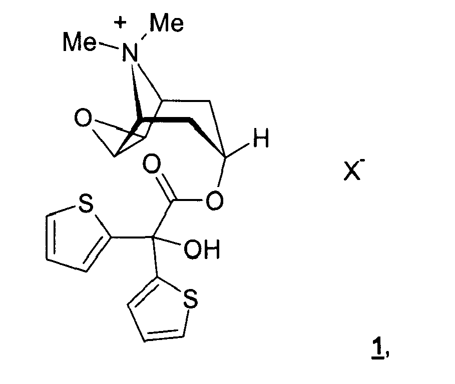 worin
   X ⁻ ein negativ geladenes Anion bedeutet.
ii) mindestens einem Einbettungsmaterial ausgewählt aus der Gruppe bestehend aus Mono- oder Disacchariden, Oligosacchariden, Polymeren, Zuckeralkoholen und Cholesterin.
verwendet.

Dieses zusätzliche Salz einer organischen, physiologisch unbedenklichen, sterisch anspruchsvollen Säure wird hierbei vorzugsweise in einer solchen Konzentration eingesetzt, dass sich ein molares Verhältnis des Anticholinergikums nach i), insbesondere der Verbindung nach Formel 1, zum Kation des Salzes von 1:1 bis 1:12, vorzugsweise von 1:2 bis 1:10 und insbesondere von 1:3 bis 1:8 ergibt.

In einer anderen besonders bevorzugten Ausführungsform wird ein Gemisch einer organischen, physiologisch unbedenklichen, sterisch anspruchsvollen Säure nach iii) vorzugsweise ausgewählt aus der Gruppe bestehend aus Ascorbinsäure, einer ein-, zwei- oder dreiwertigen Carbonsäure mit Ausnahme der Aminocarbonsäuren, vorzugsweise Fumarsäure, Oxalsäure oder Bernsteinsäure, und einer Frucht- oder Genusssäure, vorzugsweise Citronensäure, Weinsäure, Äpfelsäure, Milchsäure, Essigsäuret, α-Hydroxycaprylsäure oder Gluconsäure
und dem Salz einer organischen, physiologisch unbedenklichen, sterisch anspruchsvollen Säure nach iii)
vorzugsweise ausgewählt aus der Gruppe bestehend aus Ascorbat, dem Salz einer ein-, zwei- oder dreiwertigen Carbonsäure mit Ausnahme der Salze der Aminocarbonsäuren, vorzugsweise Fumarat, Oxalat oder Succinat, und dem Salz einer Frucht- oder Genusssäure, vorzugsweise Citrat, Tartrat, Malat, Lactat, Acetat, α-Hydroxycapronat oder Gluconat,
zur Stabilisierung einer mittels Sprühtrocknung hergestellten Pulverformulierung enthaltend die folgenden Komponenten i) bis ii):
i) eine Verbindung der Formel **1** 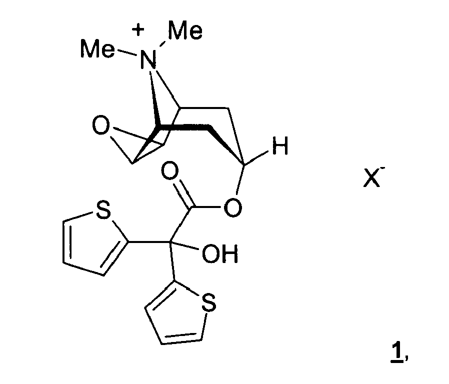 worin
   X ⁻ ein negativ geladenes Anion bedeutet, und
ii) mindestens ein Einbettungsmaterial ausgewählt aus der Gruppe bestehend aus Mono- oder Disacchariden, Oligosacchariden, Polymeren, Zuckeralkoholen und Cholesterin.
verwendet.
Besonders bevorzugt ist hierbei die Verwendung eines Gemisches aus Citronensäure und Citrat, vorzugsweise einem Alkalimetallcitrat, einem Erdalkalimetallcitrat oder Zinkcitrat, insbesondere Natriumcitrat, Kaliumcitrat, Magnesiumcitrat, Calciumcitrat oder Zinkcitrat.

Vorzugsweise wird das Verhältnis der organischen, physiologisch unbedenklichen, sterisch anspruchsvollen Säure, vorzugsweise Citronensäure, zum Salz der organischen, physiologisch unbedenklichen, sterisch anspruchsvollen Säure, vorzugsweise Citrat, in der Lösung derart eingestellt, dass die in Schritt a) hergestellte Lösung einen pH von < 7, vorzugsweise von ≤6, stärker bevorzugt von ≤5, insbesondere von ≤4 besitzt.

Vorzugsweise wird eine solche Konzentration des Salzes der organischen, physiologisch unbedenklichen, sterisch anspruchsvollen Säure eingesetzt, dass sich ein molares Verhältnis des Anticholinergikums nach i), insbesondere der Verbindung nach Formel **1**, zum Kation des Salzes von 1:1 bis 1:12, vorzugsweise von 1:2 bis 1:10 und insbesondere von 1:3 bis 1:8 ergibt.

Vorzugsweise bedeutet X ⁻ der Verbindung der Formel **1** ein Anion ausgewählt aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, insbesondere Bromid.

Vorzugsweise wird als Einbettungsmaterial ii) ein Mono- oder Disaccharid ausgewählt aus der Gruppe bestehend aus Glucose, Saccharose, Fructose, Maltose, Lactose, Cellobiose und Trehalose, ein Oligosaccharid ausgewählt aus der Gruppe Oligomaltose, Oligofructose, Cyclodextrine, Dextrine und Oligosaccharose, ein Polymer ausgewählt aus der Gruppe Inulin, Alginat, Maltodextrin, Stärke, Stärkederivate, Cellulose, Cellulosederivate, PVP (Plasdone), Gelatine, Chitosan, Dextrane, Pektine, Gummi Arabicum, Polylaktide, Poly(laktid-co-glykolide) und Polyvinylalkohole, ein Zuckeralkohol ausgewählt aus der Gruppe Mannitol, Xylitol und Sorbitol oder Cholesterin eingesetzt. Besonders bevorzugt als Einbettungsmaterial ii) sind jedoch Lactose, Trehalose und Mannitol, insbesondere Lactose.

Die vorliegende Erfindung betrifft ferner die Verwendung der erfindungsgemäßen sprühgetrockneten Pulverformulierung zur Herstellung eines Arzneimittels zur Behandlung von Atemwegserkrankungen, insbesondere zur Behandlung von COPD und/oder Asthma, wobei vorzugsweise die vorstehend beschriebenen Inhalatoren zur Anwendung gelangen.

### BEISPIELE

### Formulierungsbeispiele:

### Lösungen nach Schritt a) mit Citronensäure:

| Lösung vor der Sprühtrocknung: | |
|---|---|
| Verbindung nach Formel **1** (X ⁻ = BR ⁻) | 0,4 g (1% bez. auf den Feststoff) |
| Lactose | 40,6 g |
| Citronensäure | 0,25 g (Zugabe bis pH 3; |
| | 0,6% bezogen auf den Gesamt-Feststoff der Partikel) |
| Wasser | 460 g |

### Lösungen nach Schritt a) mit Citronensäure und Citrat:

| Lösung vor der Sprühtrocknung: | |
|---|---|
| Verbindung nach Formel **1** (X ⁻ = BR ⁻) | 0,4 g (1% bez. auf den Feststoff) |
| Lactose | 38,5 g |
| Citronensäure | 2,1 g (Zugabe bis pH 3; |
| | 5,2% bezogen auf den Gesamt-Feststoff der Partikel) |
| Zinkcitrat Dihydrat | 1,0 g (2,5% bezogen auf den Gesamt-Feststoff der Partikel) molares Verhältnis des Anticholinergikums zum Zink-lon beträgt 1:6 |
| Wasser | 460 g |

### Herstellungsbeispiele:

### Sprühgetrocknete Pulverformulierung (mit Citronensäure):

### Beispiel 1: Sprühparameter, geeignet zur Erzeugung inhalierbarer Partikel mit Ba 679

### Durchführung:

In einem Erlenmeyerkolben wird das Lösungsmittel vorgelegt. Die Zugabe des Einbettungsmaterial erfolgt portionsweise unter starkem Rühren (z.B. Magnetrührer) und ggf. unter Erwärmung. Sobald die Lösung klar ist, erfolgt die Einstellung des pH-Wertes mit Citronensäure auf pH = 3 und die Zugabe der Verbindung nach Formel **1**. Nach vollständigem Lösen schließt sich umgehend die Sprühtrocknung an.
Die Sprühtrocknung erfolgt mittels einem modifizierten BÜCHI Mini-Spray Dryer (B-191) in Verbindung mit einer modifizierten 0,5 mm Zwei-Stoff-Düse und unter der ausschließlichen Verwendung von N₂ als Prozess- und Düsengas. Im Wesentlichen wurden hierbei alle Glasteile durch Metallteile ersetzt und der Aspirator entfernt. Über den Prozessgaseintritt wird N₂ als Trockengas zugeführt (ca. 35 m³/h) so dass das Gerät im Überdruckbereich durchströmt wird. Der Ausgangsfilter zwischen Zyklon und Aspirator wurde entfernt und der Gasaustritt direkt nach dem Zyklon in einen Abzug mit integriertem Partikelfeinfilter geleitet. Die Zweistoffdüse wurde aus Edelstahl gefertigt, wobei die 0,5 mm Düsenkappe mit Mischnadel und Düsenüberwurfmutter als zentrale Zerstäubungseinheit beibehalten wurden. Der Massenstrom des Düsengasdurchsatzes wird über ein externes Messgerät (Kobold MAS 3015) ermittelt und von dem ursprünglichen Schwebekörperdurchflussmesser entkoppelt. Üblicherweise wird die Düse bei einem Gasdruck von ca. 6 bar Überdruck betrieben. Die Eingangstemperatur des Prozessgases beträgt 150°C. Der Massenstrom der Sprühlösung ist derart zu wählen, dass eine Ausgangstemperatur von 82±3°C gegeben ist. Nach erfolgter Sprühtrocknung ist das Pulver umgehend zu entnehmen und unter Ausschluss von Feuchtigkeit zu Lagern bzw. weiter zu verarbeiten. Die verwendeten Prozessparameter sind in Tabelle 1 ersichtlich.

**Tabelle 1. Sprühtrocknungsparameter**

| | |
|---|---|
| Volumenstrom "Sprührate" | 12 ml/min |
| Sprühdruck (Düsentyp) | 6 bar Überdruck N₂ (BÜCHI Sprühdüse 0.5 mm, modifiziert) |
| Volumenstrom "Zerstäubungsdruck" (Düsentyp) | 2580 Normliter / h (BÜCHI Sprühdüse 0.5 mm, modifiziert) |
| Eingangstemperatur | 150°C |
| Ausgangstemperatur | 80°C |
| Volumenstrom "Trocknungsgas" | 31 Norm m³ / h |
| Querschnitt Trockenturm | 105 mm |

### Sprühgetrocknete Pulverformulierung (mit Citronensäure und Citrat):

### Beispiel 2: Sprühparameter, geeignet zur Erzeugung inhalierbarer Partikel mit Ba 679 Durchführung:

In einem Erlenmeyerkolben wird das Lösungsmittel vorgelegt. Die Zugabe des Einbettungsmaterial erfolgt portionsweise unter starkem Rühren (z.B. Magnetrührer) und ggf. unter Erwärmung. Sobald die Lösung klar ist, erfolgt die Zugabe des Salzes einer organischen Säure und die Einstellung des pH-Wertes mit Citronensäure auf pH = 3. Anschließend erfolgt die Zugabe der Verbindung nach Formel 1. Nach vollständigem Lösen schließt sich umgehend die Sprühtrocknung an.
Die Sprühtrocknung erfolgt wie unter Beispiel 1 beschrieben. Die verwendeten Prozessparameter sind in Tabelle 2 ersichtlich.

**Tabelle 2. Sprühtrocknungsparameter**

| | |
|---|---|
| Volumenstrom "Sprührate" | 12 ml/min |
| Sprühdruck (Düsentyp) | 6 bar Überdruck N2 (BÜCHI Sprühdüse 0.5 mm, modifiziert) |
| Volumenstrom "Zerstäubungsdruck" (Düsentyp) | 2580 Normliter / h (BÜCHI Sprühdüse 0.5 mm, modifiziert) |
| Eingangstemperatur | 150°C |
| Ausgangstemperatur | 79°C |
| Volumenstrom "Trocknungsgas" | 31 Norm m3 / h |
| Querschnitt Trockenturm | 105 mm |

## Patentansprüche

1. Sprühgetrocknete Pulverformulierung umfassend Partikel, die die folgenden Komponenten i) bis iii) enthalten:
i) eine Verbindung der Formel **1** 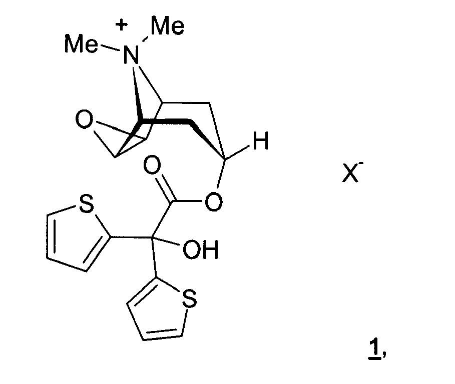 worin
X ⁻ ein negativ geladenes Anion bedeutet.
ii) mindestens einem Einbettungsmaterial ausgewählt aus der Gruppe bestehend aus Mono- oder Disacchariden, Oligosacchariden, Polymeren, Zuckeralkoholen und Cholesterin,
iii) eine organische, physiologisch unbedenkliche, sterisch anspruchsvolle Säure ausgewählt aus der Gruppe bestehend aus Ascorbinsäure, einer Frucht- oder Genusssäure und einer ein-, zwei- oder dreiwertigen Carbonsäure mit Ausnahme der Aminocarbonsäuren.

2. Sprühgetrocknete Pulverformulierung nach Anspruch 2, wobei die organische, physiologisch unbedenkliche, sterisch anspruchsvolle Säure nach iii) eine Frucht- oder Genusssäure ausgewählt aus der Gruppe bestehend aus Citronensäure, Weinsäure, Äpfelsäure, Milchsäure, Essigsäure, α-Hydroxycaprylsäure und Gluconsäure oder eine ein-, zwei- oder dreiwertige Carbonsäure, mit Ausnahme der Aminocarbonsäuren, ausgewählt aus der Gruppe bestehend aus Fumarsäure, Oxalsäure und Bernsteinsäure ist.

3. Sprühgetrocknete Pulverformulierung nach Anspruch 1 oder 2, wobei die organische, physiologisch unbedenkliche, sterisch anspruchsvolle Säure nach iii) Citronensäure ist.

4. Sprühgetrocknete Pulverformulierung nach einem der Ansprüche 1 bis 3, wobei die sprühgetrocknete Pulverformulierung zusätzlich das Salz einer organischen, physiologisch unbedenklichen, sterisch anspruchsvollen Säure nach iii) ausgewählt aus der Gruppe bestehend aus Ascorbat, dem Salz einer Frucht- oder Genusssäure und dem Salz einer ein-, zwei- oder dreiwertigen Carbonsäure, mit Ausnahme der Salze der Aminocarbonsäuren, enthält.

5. Sprühgetrockenete Pulverformulierung nach einem der Ansprüche 1 bis 4, wobei die sprühgetrocknete Pulverformulierung zusätzlich das Salz einer organischen, physiologisch unbedenklichen, sterisch anspruchsvollen Säure nach iii) ausgewählt aus der Gruppe bestehend aus dem Salz einer Frucht- oder Genusssäure ausgewählt aus Citrat, Tartrat, Malat, Lactat, Acetat, α-Hydroxycapronat und Gluconat oder dem Salz einer ein-, zwei- oder dreiwertigen Carbonsäure, mit Ausnahme der Salze der Aminocarbonsäuren, ausgewählt aus der Gruppe bestehend aus Fumarat, Oxalat und Succinat, enthält.

6. Sprühgetrockente Pulverformulierung nach Anspruch 5, wobei die sprühgetrocknete Pulverformulierung als Salz der organischen, physiologisch unbedenklichen, sterisch anspruchsvollen Säure nach iii) Citrat enthält.

7. Sprühgetrockente Pulverformulierung nach einem der Ansprüche 5 oder 6, wobei das Salz der organischen, physiologisch unbedenklichen, sterisch anspruchsvollen Säure ein Alkalimetallsalz, ein Erdalkalimetallsalz oder ein Zinksalz ist.

8. Sprühgetrocknete Pulverformulierung nach einem der Ansprüche 1 bis 7, wobei in der Verbindung nach Formel **1** nach i) X ⁻ ein Anion ausgewählt aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat bedeutet.

9. Sprühgetrocknete Pulverformulierung nach Anspruch 8, wobei in der Verbindung nach Formel **1** nach i) X ⁻ Bromid bedeutet.

10. Sprühgetrocknete Pulverformulierung nach einem der Ansprüche 1 bis 9, wobei die Verbindung nach Formel **1** in einer Konzentration zwischen 0,01 bis 5 % bezogen auf den Feststoff vorliegt.

11. Sprühgetrocknete Pulverformulierung nach Anspruch 10, wobei die Verbindung nach Formel **1** in einer Konzentration zwischen 0,02 bis 2 % bezogen auf den Feststoff vorliegt.

12. Sprühgetrocknete Pulverformulierung nach einem der Ansprüche 1 bis 11, wobei das Einbettungsmaterial nach ii) ein Mono- oder Disaccharid ausgewählt aus der Gruppe Glucose, Saccharose, Fructose, Maltose, Lactose, Cellobiose und Trehalose ist.

13. Sprühgetrocknete Pulverformulierung nach einem der Ansprüche 1 bis 11, wobei das Einbettungsmaterial nach ii) ein Oligosaccharid ausgewählt aus der Gruppe Oligomaltose, Oligofructose, Cyclodextrine, Dextrine und Oligosaccharose ist.

14. Sprühgetrocknete Pulverformulierung nach einem der Ansprüche 1 bis 11, wobei das Einbettungsmaterial nach ii) ein Polymer ausgewählt aus der Gruppe Inulin, Alginat, Maltodextrin, Stärke, Stärkederivate, Cellulose, Cellulosederivate, PVP, Gelatine, Chitosan, Dextrane, Pektine, Gummi Arabicum, Polylaktide, Poly(laktid-co-glykolide) und Polyvinylalkohole ist.

15. Sprühgetrocknete Pulverformulierung nach einem der Ansprüche 1 bis 11, wobei das Einbettungsmaterial nach ii) ein Zuckeralkohol ausgewählt aus der Gruppe Mannitol, Xylitol und Sorbitol ist.

16. Sprühgetrocknete Pulverformulierung nach einem der Ansprüche 1 bis 15, wobei die organische, physiologisch unbedenkliche, sterisch anspruchsvolle Säure nach iii) Citronensäure ist und die Citronensäure in einer solchen Konzentration zu der sprühfertigen Lösung enthaltend das Anticholinergikum nach i), vorzugsweise eine Verbindung nach Formel **1**, und das mindestens eine Einbettungsmaterial nach ii) gegeben wird, dass sich ein pH der Lösung von ≤6 ergibt.

17. Sprühgetrocknete Pulverformulierung nach Anspruch 16, wobei die Citronensäure in einer solchen Konzentration zu der sprühfertigen Lösung zugegeben wird, dass sich ein pH der Lösung von ≤5 ergibt.

18. Sprühgetrocknete Pulverformulierung nach einem der Ansprüche 1 bis 17, wobei die Partikel einen mittleren aerodynamischen Durchmesser von maximal 15 µm, vorzugsweise von maximal 10 µm besitzen.

19. Sprühgetrocknete Pulverformulierung nach Anspruch 18, wobei die Partikel inhalierbar sind.

20. Verfahren zur Herstellung einer Pulverformulierung, die Partikel enthält, umfassend die folgenden Schritte:
a) Bereitstellen einer Lösung umfassend die Komponenten i) bis iii):
i) eine Verbindung der Formel **1** 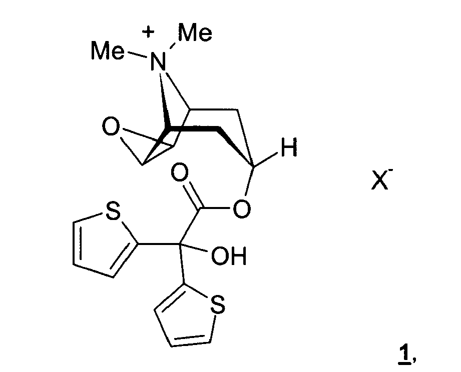 worin
X ⁻ ein negativ geladenes Anion bedeutet,
ii) mindestens ein Einbettungsmaterial ausgewählt aus der Gruppe bestehend aus Mono- oder Disacchariden, Oligosacchariden, Polymeren, Zuckeralkoholen und Cholesterin, und
iii) eine organische, physiologisch unbedenkliche, sterisch anspruchsvolle Säure ausgewählt aus der Gruppe bestehend aus Ascorbinsäure, einer Frucht- oder Genusssäure und einer ein-, zwei- oder dreiwertigen Carbonsäure mit Ausnahme der Aminocarbonsäuren
in einem geeigneten Lösungsmittel;
b) Zerstäuben der Lösung aus Schritt a) in einem Heißluftstrom
c) Trocknen der zerstäubten Lösung aus Schritt b) zu einem Pulver enthaltend Partikel.
d) Abscheidung des Pulvers aus Schritt c) mittels Zyklon oder Filters.

21. Verfahren nach Anspruch 20, worin in Schritt a) eine Lösung bereitgestellt wird, die als organische, physiologisch unbedenkliche, sterisch anspruchsvolle Säure nach iii) eine Frucht- oder Genusssäure ausgewählt aus der Gruppe bestehend aus Citronensäure, Weinsäure, Äpfelsäure, Milchsäure, Essigsäure, α-Hydroxycaprylsäure und Gluconsäure oder eine ein-, zwei- oder dreiwertige Carbonsäure, mit Ausnahme der Aminocarbonsäuren, ausgewählt aus der Gruppe bestehend aus Fumarsäure, Oxalsäure und Bernsteinsäure ist.

22. Verfahren nach Anspruch 20 oder 21, worin in Schritt a) eine Lösung bereitgestellt wird, die als organische, physiologisch unbedenkliche, sterisch anspruchsvolle Säure nach iii) Citronensäure enthält.

23. Verfahren nach einem der Ansprüche 20 bis 22, worin in Schritt a) eine Lösung bereitgestellt wird, die zusätzlich das Salz einer organischen, physiologisch unbedenklichen, sterisch anspruchsvollen Säure nach iii) ausgewählt aus der Gruppe bestehend aus Ascorbat, dem Salz einer Frucht- oder Genusssäure und dem Salz einer ein-, zwei- oder dreiwertigen Carbonsäure, mit Ausnahme der Salze der Aminocarbonsäuren, enthält.

24. Verfahren nach Anspruch 23, worin in Schritt a) eine Lösung bereitgestellt wird, die als zusätzliches Salz einer organischen, physiologisch unbedenklichen, sterisch anspruchsvollen Säure nach iii) das Salz einer Frucht- oder Genusssäure ausgewählt aus Citrat, Tartrat, Malat, Lactat, Acetat, α-Hydroxycapronat und Gluconat oder das Salz einer ein-, zwei- oder dreiwertigen Carbonsäure, mit Ausnahme der Salze der Aminocarbonsäuren, ausgewählt aus der Gruppe bestehend aus Fumarat, Oxalat und Succinat, enthält.

25. Verfahren nach Anspruch 24, worin in Schritt a) eine Lösung bereitgestellt wird, die zusätzlich als Salz einer organischen, physiologisch unbedenklichen, sterisch anspruchsvollen Säure nach iii) Citrat, vorzugsweise ein Alkalimetallcitrat, ein Erdalkalimetallcitrat oder Zinkcitrat, enthält.

26. Verfahren nach einem der Ansprüche 20 bis 25, wobei in Schritt a) eine Lösung bereitgestellt wird, die eine Verbindung der Formel **1** enthält, worin X ⁻ ein Anion ausgewählt aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat bedeutet.

27. Verfahren nach einem der Ansprüche 20 bis 26, wobei in Schritt a) eine Lösung bereitgestellt wird, die eine Verbindung der Formel **1** umfasst, worin X ⁻Bromid ist.

28. Verfahren nach einem der Ansprüche 20 bis 27, wobei das Einbettungsmaterial ii) der Lösung aus Schritt a) ein Mono- oder Disaccharid ausgewählt aus der Gruppe bestehend aus Glucose, Saccharose, Fructose, Maltose, Lactose, Cellobiose und Trehalose ist oder ein Oligosaccharide ausgewählt aus der Gruppe bestehend aus Oligomaltose, Oligofructose, Cyclodextrine, Dextrine und Oligosaccharose ist oder ein Polymer ausgewählt aus der Gruppe bestehend aus Inulin, Alginat, Maltodextrin, Stärke, Stärkederivate, Cellulose, Cellulosederivate, PVP, Gelatine, Chitosan, Dextrane, Pektine, Gummi Arabicum, Polylaktide, Poly(laktid-co-glykolide) und Polyvinylalkohole ist oder ein Zuckeralkohol ausgewählt aus der Gruppe bestehend aus Mannitol, Xylitol und Sorbitol oder Cholesterin ist.

29. Verfahren nach einem der Ansprüche 20 bis 28, wobei die Partikel aus Schritt c) einen mittleren Durchmesser von maximal 15 µm, vorzugsweise von maximal 10 µm besitzen.

30. Verfahren nach einem der Ansprüche 20 bis 29, wobei die Partikel aus Schritt c) inhalierbar sind.

31. Verfahren nach einem der Ansprüche 20 bis 30, wobei das Zerstäuben nach Schritt b) mittels einer Düse erfolgt.

32. Verfahren nach einem der Ansprüche 20 bis 31, wobei der Heißluftstrom nach Schritt b) Temperaturen zwischen 100 und 350°C, vorzugsweise zwischen 120 und 200 °C besitzt.

33. Pulverformulierung erhältlich durch ein Verfahren nach einem der Ansprüche 20 bis 32.

34. Verwendung einer organischen, physiologisch unbedenklichen, sterisch anspruchsvollen Säure ausgewählt aus der Gruppe bestehend aus Ascorbinsäure, einer Frucht- oder Genusssäure und einer ein-, zwei- oder dreiwertigen Carbonsäure, mit Ausnahme der Aminocarbonsäuren, zur Stabilisierung einer mittels Sprühtrocknung hergestellten Pulverformulierung enthaltend die folgenden Komponenten i) bis ii):
i) eine Verbindung der Formel **1** 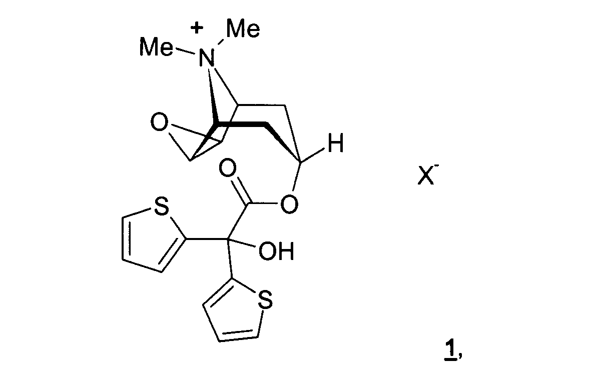 worin
X ⁻ ein negativ geladenes Anion bedeutet, und
ii) mindestens ein Einbettungsmaterial ausgewählt aus der Gruppe bestehend aus Mono- oder Disacchariden, Oligosacchariden, Polymeren, Zuckeralkoholen und Cholesterin.

35. Verwendung nach Anspruch 34, wobei die organische, physiologisch unbedenkliche, sterisch anspruchsvolle Säure eine Frucht- oder Genusssäure ausgewählt aus der Gruppe bestehend aus Citronensäure, Weinsäure, Äpfelsäure, Milchsäure, Essigsäure, α-Hydroxycaprylsäure und Gluconsäure oder eine ein-, zwei- oder dreiwertige Carbonsäure, mit Ausnahme der Aminocarbonsäuren, ausgewählt aus der Gruppe bestehend aus Fumarsäure, Oxalsäure und Bernsteinsäure ist.

36. Verwendung nach Anspruch 34 oder 35, wobei die organische, physiologisch unbedenkliche, sterisch anspruchsvolle Säure Citronensäure ist

37. Verwendung eines Gemisches einer organischen, physiologisch unbedenklichen, sterisch anspruchsvollen Säure ausgewählt aus der Gruppe bestehend aus Ascorbinsäure, einer Frucht- oder Genusssäure und einer ein-, zwei- oder dreiwertigen Carbonsäure, mit Ausnahme der Aminocarbonsäuren, und dem Salz einer organischen, physiologisch unbedenklichen, sterisch anspruchsvollen Säure ausgewählt aus der Gruppe bestehend aus Ascorbat, dem Salz einer Frucht- oder Genusssäure und dem Salz einer ein-, zwei- oder dreiwertigen Carbonsäure, mit Ausnahme der Salze der Aminocarbonsäuren, zur Stabilisierung einer mittels Sprühtrocknung hergestellten Pulverformulierung enthaltend die folgenden Komponenten i) bis ii):
i) eine Verbindung der Formel **1** 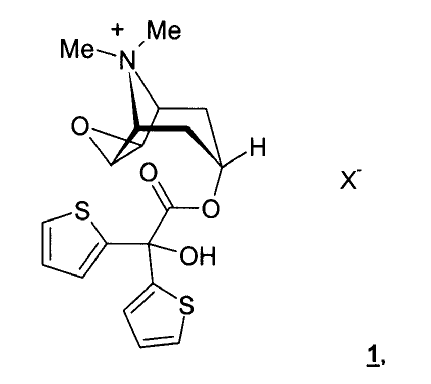 worin
X ⁻ ein negativ geladenes Anion bedeutet.
ii) mindestens einem Einbettungsmaterial ausgewählt aus der Gruppe bestehend aus Mono- oder Disacchariden, Oligosacchariden, Polymeren, Zuckeralkoholen und Cholesterin.

38. Verwendung eines Gemisches nach Anspruch 37, wobei die organische, physiologisch unbedenkliche, sterisch anspruchsvolle Säure eine Frucht- oder Genusssäure ausgewählt aus der Gruppe bestehend aus Citronensäure, Weinsäure, Äpfelsäure, Milchsäure, Essigsäure, α-Hydroxycaprylsäure und Gluconsäure oder eine ein-, zwei- oder dreiwertige Carbonsäure, mit Ausnahme der Aminocarbonsäuren, ausgewählt aus der Gruppe bestehend aus Fumarsäure, Oxalsäure und Bernsteinsäure ist und wobei das Salz der organischen, physiologisch unbedenklichen, sterisch anspruchsvollen Säure das Salz einer Frucht- oder Genusssäure ausgewählt aus der Gruppe bestehend aus Citrat, Tartrat, Malat, Lactat, Acetat, α-Hydroxycapronat und Gluconat oder das Salz einer ein-, zwei- oder dreiwertigen Carbonsäure, mit Ausnahme der Salze der Aminocarbonsäuren, ausgewählt aus der Gruppe bestehend aus Fumarat, Oxalat und Succinat, ist.

39. Verwendung nach Anspruch 38, wobei als organische, physiologisch unbedenkliche, sterisch anspruchsvolle Säure Citronensäure und als Salz einer organischen, physiologisch unbedenklichen, sterisch anspruchsvollen Säure Citrat, vorzugsweise ein Alkalimetallcitrat, ein Erdalkalimetallcitrat oder Zinkcitrat, eingesetzt wird.
